# EUROPEAN PATENT APPLICATION

(11) **EP 3 725 365 A1**
(43) Date of publication of application: **21.10.2020**
(21) Application number: 19169408.2
(22) Date of filing: 16.04.2019
(51) Int. Cl.: A61N 1/375, C23C 4/129, C23C 4/134, H01M 2/06

(54) **FEEDTHROUGH COMPONENT WITH PLASMA CERAMIC INCLUDING CRYSTALLIZED GLASS SOLDER**

(71) Applicant: BIOTRONIK SE & Co. KG, 12359 Berlin (DE)
(72) Inventor: Sontheimer, Thomas, 90574 Roßtal (DE)
(74) Representative: Keck, Hans-Georg

(57) **Abstract**

The present invention relates to a feedthrough for an electric component or electric device, particularly an implantable medical device, comprising at least one electrically conductive connecting element and an electrically conductive flange with at least one passage configured to receive the at least one conductive connecting element, wherein the at least one electrically conductive connecting element or the electrically conductive flange comprises a first layer made of a thermally sprayed ceramic and a gas-tight second layer made of glass or ceramic, and the first layer is at least partly, particularly completely, covered by the second layer. The present invention further relates to a method of manufacturing the feedthrough of the invention, and an electric device or component comprising the feedthrough of the invention.

## Description

The present invention relates to a feedthrough, particularly for an implantable medical device, a method for manufacture thereof.

Electrical feedthroughs, particularly for implantable medical devices, are known. They serve to guide electrical contacts insulated from the housing of an implant to the outside, while maintaining the hermetic seal of the implant.

Commonly, such electrical feedthrough comprises a flange made of the same material as the housing of the implant, e.g. titanium or a titanium alloy, and a connecting pin, with which an electrical contact is established between the components within the hermetically sealed interior of the implant and the surrounding of the implant. The connecting pin is usually comprised by an insulator, preferably a ceramic or glass solder, wherein the insulator is connected to the flange. Typically, such feedthrough comprises one or more connecting pins. Presently, the aforementioned components (flange, connecting pins) are joined via brazing to yield the feedthrough, wherein a comparable large insulation body is inserted into a flange made of metal and brazed with gold.

One major drawback of existing feedthrough manufacturing techniques is the increasingly limited technical feasibility of the manufacturing of oxidic ceramics, particularly in terms of shape tolerance and/or positional tolerance. Furthermore, the ceramic insulation body frequently is the weak spot in an electric feedthrough. For example, a failure of the ceramic (crack or breach) is most cases the cause for insufficient tightness (He tightness) due to thermal stress during welding the housing of the implant.

Particularly, this weakness of common ceramic insulation bodies encumbers the manufacturing process, particularly the brazing process with high temperatures, and increases thereby increases the costs of both the component and the manufacturing process.

Based on this background, it is an objective of the present invention to provide feedthrough components that are robust and more simple to process, particularly with regard to the joining process.

The above objective is attained by a feedthrough having the features of claim 1, an electric device having the features of claim 10, an electric component having the features of claim 11 and a method for manufacturing a feedthrough having the features of claim 12. Preferred embodiments are stated in the respective dependent claims and the following description.

According to claim 1, an electric feedthrough is provided, which comprises at least one electrically conductive connecting element and an electrically conductive flange with at least one passage configured to receive the at least one conductive connecting element, wherein
- the at least one electrically conductive connecting element or the electrically conductive flange comprises a first layer made of a thermally sprayed ceramic, particularly a plasma ceramic, and a gas-tight second layer made of glass or ceramic, and
- the first layer is at least partly, particularly completely, surrounded by the second layer.

The term "thermally sprayed ceramic" in the context of the present specification particularly refers to a ceramic that is manufactured by thermal spraying, particularly flame or plasma spraying, wherein an oxidic ceramic powder is heated to or above the melting temperature and propelled or sprayed towards a surface to be coated with the oxidic ceramic. Particularly, such thermally sprayed ceramic, particularly a plasma ceramic, is characterized by an increased porosity, e.g. in the range of approx. 1 vol. % to approx. 10 vol. %. Preferred are plasma ceramics manufactured by plasma spraying utilizing a water-stabilized plasma torch.

The use of a thermally sprayed ceramic, particularly a plasma ceramic, as insulating material has several advantages:
- Thermally sprayed ceramics, particularly plasma ceramics, are characterized by a thermal stability up to 1300°C. Accordingly, an insulating body made of a plasma ceramic is more resistant against thermal stress, which could be occur, e.g. during brazing of the connecting element into the flange or welding the flange into the housing of an implantable medical device.
- An insulting body made of a thermally sprayed ceramic, particularly a plasma ceramic, may be manufactured with a smaller thickness while maintaining the desired insulating properties.

Particularly, the electrically conductive flange comprises the first layer made of a thermally sprayed ceramic, particularly a plasma ceramic, on that surface area that forms the at least one passage and that is intended to be joined with the electrically conductive connecting element.

In one embodiment of the feedthrough of the specification, the at least one electrically conductive connecting element is arranged, particularly fixed, in the at least one passage of the electrically conductive flange. Preferably, the at least one electrically conductive element is joined with the electrically conductive flange such that the feedthrough is hermetically sealed. Preferable the at least one conductive element is joined by brazing, particularly using gold as a solder.

In a further embodiment of the feedthrough of the invention, the second layer is at least partly covered by a third layer made of metal, wherein particularly said third layer comprises or consists of Nb, Pt, Pt/Ir, FeNi, FeNiCo, FeCr, Ta, Mo, W, Cr, Co, V, Ti or an alloy thereof.

In a further embodiment of the feedthrough of the invention, the thermally sprayed ceramic particularly the plasma ceramic, consists of or comprises Al₂O₃, Al₂TiO₅, TiO₂, MgO, CaO, Na₂O, K₂O, Nb₂O₅, ZrO₂, ZrO₂*SiO₂, Y₂O₃, 3Al₂O₃*2SiO2, 2Al₂O₃*SiO₂, MgAl₂O₄ or a mixture thereof.

In a further embodiment of the feedthrough of the invention, the second layer consists of or comprises SiO₂, ZrSiO₂, Na₂O, K₂O, Al₂O₃, MgO, CaO , BaO or a mixture thereof, particularly SiO₂-B₂O₃-Na₂O-K₂O-Al₂O₃-MgO-CaO (particularly know as borosilicate glass).

In a further embodiment of the feedthrough of the invention, the second layer is characterized by an amorphous structure or a crystalline structure.

The term "amorphous structure" or "non-crystalline structure" in the context of the present invention is used in its meaning known in the art of material science; it particularly refers to a structure that lacks long-range order that is characteristic of a crystal. A non-limiting example for a substance or solid with an amorphous structure is plain glass.

The term "crystalline structure" in the context of the present invention is used in its meaning known in the art of material science; it particularly refers to a highly order microscopic structure along which the constituents of a respective solid are arranged and which forms a crystal lattice that extends in all directions. A non-limiting example for a crystalline solid is quartz.

In a further embodiment of the feedthrough of the invention, the at least one electrically conductive connecting element consists of or comprises a metal or metal alloy, particularly Nb, Pt, Pt/Ir, FeNi, FeNiCo, FeCr, Ta, Mo, W, Cr, V, Ti or an alloy thereof, particularly Ti₆Al₄V.

In a further embodiment of the feedthrough of the invention, the flange of made of a biocompatible material, particularly a biocompatible metal or metal alloy, more particular titanium or a titanium alloy such as for example Ti₆Al₄V.

In a further embodiment of the feedthrough of the invention, the first layer is characterized by a thickness in the range of 100 µm to 500 µm, particularly in the range of 150 µm to 300 µm.

According to claim 10, an electric device is provided, wherein the device comprises a feedthrough according to claim 1 or any embodiment thereof.

In one embodiment of the electric device of the invention, the electric device is an implantable medical device, which comprises a hermetically sealed housing comprising at least one electric or electronic components such as, for example, an electronic module, a power source, particularly a battery and/or a capacitor, wherein the feedthrough of the invention is arranged, particularly welded, in the housing, and the connecting element is in an electrical contact with the at least one electric or electronic module. Particularly, the interior of the implantable medical device defined by the housing is hermetically sealed from the outside by the housing and the feedthrough.

In a further embodiment of the electric device of the invention, the housing of the implantable medical device is made of biocompatible metal or a metal alloy, particularly titan or a titan alloy such as, for example Ti₆Al₄V, and the feedthrough of the invention is welded into the housing.

In a further embodiment of the electric device of the invention, the device is selected from a pacemaker, a cardioverter defibrillator, and a neurostimulator.

According to claim 11, an electric component is provided, wherein the electric component comprises a feedthrough according to claim 1 or any embodiment thereof.

In one embodiment of the electric component of the invention, the electric component is a battery, a capacitor, or a printed circuit board.

In a further embodiment of the electric component of the invention, the electric component comprises a housing comprising at least one electric or electronic element, wherein the connecting element of the feedthrough of the invention is in electrical contact with the at least one electric or electronic element.

In a further embodiment of the electric component of the invention, the electric or electronic component is a cathode, an anode or an integrated circuit.

According to claim 12, a method for manufacturing a feedthrough, particularly a feedthrough according to claim 1 or any embodiment thereof, is provided. The method comprises the steps of:
- providing an electrically conductive connecting element or an electrically conductive flange with at least one passage configured to receive the electrically conductive connecting element;
- applying a ceramic powder, particularly an oxidic ceramic powder, on a surface portion of the electrically conductive connecting element or the electrically conductive flange by thermal spraying, thereby yielding a thermally sprayed ceramic layer, particularly a plasma ceramic layer;
- applying a suspended solution comprising a glazing component selected from a ceramic oxide or a glass former at least partly on said thermally sprayed ceramic layer, and
- heating the electrically conductive connecting element or the electrically conductive flange to or above the melting temperature of the glazing component, thereby yielding a gas-tight glaze layer.

The term "thermal spraying" in the context of the present specification is used in its meaning known in the art; it particularly refers to a coating process, in which melted particles, particularly melted oxidic ceramic particles, are sprayed onto a surface.

Alternatively, the ceramic powder may be applied to the electrically conductive connecting element or to the electrically conductive flange by reactive sputtering. In this case, the ceramic powder may comprise or consists of silcon nitride.

In one embodiment of the method of the invention, the oxidic ceramic powder comprises or consists of Al₂O₃, Al₂TiO₅, TiO₂, ZrO₂, ZrO₂*SiO₂, Y₂O₃, 3Al₂O₃*2SiO2, 2Al₂O₃*SiO₂, MgAl₂O₄ or a mixture thereof.

In a further embodiment of the method of the invention, the suspended solution comprising the glazing component comprises
- a glass former selected from SiO₂, B₂O3, P₂O₅, As₂O₃, GeO₂, and Sb₂O₅; and optionally
- a flux, particularly selected from Na₂O or K₂O; and optionally
- a glass stabilizer, particularly selected from Al₂O₃ and MgO.

In a further embodiment of the method of the invention, the electrically conductive connecting element consists of or comprises a metal or metal alloy, particularly Nb, Pt, Pt/Ir, FeNi, FeNiCo, FeCr, Ta, Mo, W, Cr, V, Ti or an alloy thereof.

In a further embodiment of the method of the invention, the electrically conducting flange consists of or comprises a biocompatible metal or metal alloy, particularly titanium or a titanium alloy, particularly Ti₆Al₄V.

In a further embodiment of the method of the invention, the oxidic ceramic powder is applied on a surface portion of the electrically conductive connecting element or the electrically conductive flange by flame spraying or plasma spraying.

The term "flame spraying" in the context of the present specification is used in its meaning known in the art; it particularly refers to a coating process, in which particles, particularly oxidic ceramic particles, are melted and accelerated by a flame of torch, e.g. an acetylene-oxygen flame.

The term "plasma spraying" in the context of the present specification is used in its meaning known in the art; it particularly refers to a coating process, in which particles, particularly oxidic ceramic particles, are melted and accelerated by a plasma jet emanating from a plasma torch.

In a further embodiment of the method of the invention, the plasma spraying is conducted by a water-stabilized plasma torch, wherein the plasma is formed by water. i.e. water or water vapour is the plasma gas.

In a further embodiment of the method of the invention, the method further comprises the step of applying a metal layer onto a part of the glaze layer.

In one embodiment of the method of the invention, the metal layer comprises or consists of Nb, Pt, Pt/Ir, FeNi, FeNiCo, FeCr, Ta, Mo, W, Cr, Co, V, Ti or an alloy thereof.

In a further embodiment of the method of the invention, the method further comprises the step of joining the electrically conductive connecting element to the electrically conductive flange, wherein the electrically conductive connecting element is arranged within the at least one passage of the electrically conductive flange.

In a further embodiment of the method of the invention, the electrically conductive connecting element is brazed with the electrically conducting flange, particularly using gold as a solder.

In a further embodiment of the method of the invention, the suspended solution further comprises one or more crystal nuclei.

The term "crystal nucleus" in the context of the present specification is used in its meaning known in the art; it particularly refers to a microscopic entity with pre-existing crystal structure, on which further crystallization processes can occur or which catalyses or initiates crystallization processes.

Further advantages, features and embodiments of the present invention will be explained hereinafter with reference to the drawings, in which:
Fig. 1 shows a schematic of a feedthrough component of the invention;
Fig. 2 shows an alternative feedthrough component of the invention; and
Fig. 3 shows exemplary schema of the manufacturing method of the invention.

According to the invention, it is generally envisioned to provide plasma or flame sprayed and glass solder sealed connecting pin and to use them in electrical feedthrough for implantable medical devices.

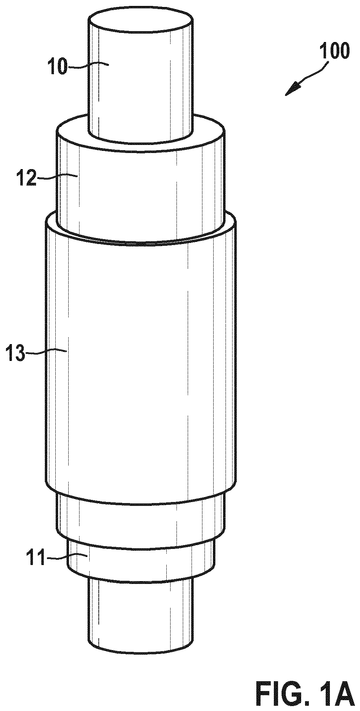

Fig. 1a shows one embodiment of the feedthrough component 100 of the invention. The component 100 comprises a connecting pin 10 made of, for example, niobium, as a connecting element for the electrical feedthrough of the invention.

The connecting pin 10 is partly covered with a first layer 11 made of a plasma ceramic, which provide the electrical insulation for the pin, while leaving the termini of the connecting pin 10 uncovered to enable an electrical connection with either the components within the housing or the header components. Accordingly, the plasma ceramic 11 is preferably formed as a hollow cylinder in case of the connecting pin is a rod or bar with circular cross-section with two edge portions. Preferably, the first layer 11 has a thickness in the range of 150 µm to 300 µm. Advantageously, the plasma ceramic 11 is characterized by a high thermal stability (up to 1300 °C).

Furthermore, the first layer 11 is at least partly, preferably completely, covered with a gas-tight second layer 12, preferably a glass solder, to seal the first layer 11 and the feedthrough, since plasma ceramics are porous and not gas-tight due to the manufacturing process. Particularly, the glass solder 12 completely covers at least one edge portion of the plasma ceramic layer and the portion of the plasma ceramic 11 which is meant to be joined into the flange, thereby effectively sealing the plasma ceramic 11 and the feedthrough.

The second layer 12 in turn is at least partly covered by a third layer 13 made of metal such as for example niobium. The third layer 13 is intended to act as a mediator for joining the connecting pin 10 or rather the feedthrough component 100 into the flange of the feedthrough of the invention. Commonly, such flange is made of the same material as the housing of an implantable device, which is usually made of titanium or a titanium alloy. Typically, the connecting pin 10, more specifically the feedthrough component 100, is brazed with a gold solder into the metallic flange, wherein the third layer 13 facilitates the brazing process.

The first 11, second 12 and third layer 13 is applied over a sufficient area of the connecting pin 10 to provide electrical insulation to the metal flange (first layer 11), gas-tightness of the feedthrough (second layer 12), and sufficient support for the brazing process (third layer 13), while leaving sufficient areas at the termini of the connecting pin 10 uncovered to provide a possibility to establish electrical contacts via the connecting pin. Preferably, the first 11, second 12 and third layer 13 are provided at least onto that surface area of the connecting pin 10, which is meant be joined into the flange by brazing.

Advantageously, by utilizing the above described plasma ceramic layer 11 instead of a common ceramic insulating body, the weakness of the latter regarding thermal stress (applied by later process steps like brazing, soldering and welding) can be overcome. At the same time, insulation with significantly reduced dimensions can be realized.

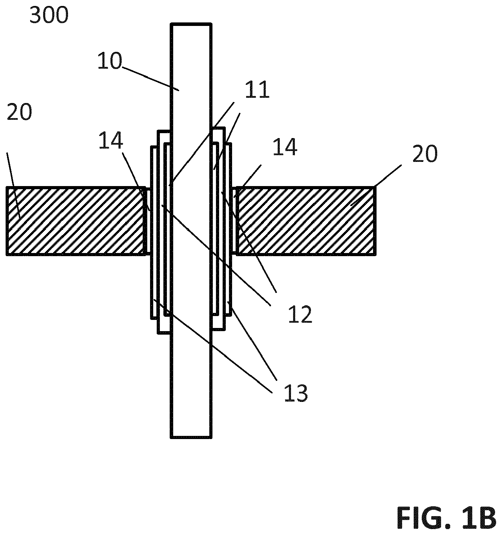

Fig. 1b illustrates the mounting condition of the above described feedthrough component 100 of the invention, which is joined to an electrically conductive flange 20, More specifically, the feedthrough component 100 is brazed into one opening of an electrically conductive flange 20 via the third layer 13 and a suitable solder 14 such as a gold solder.

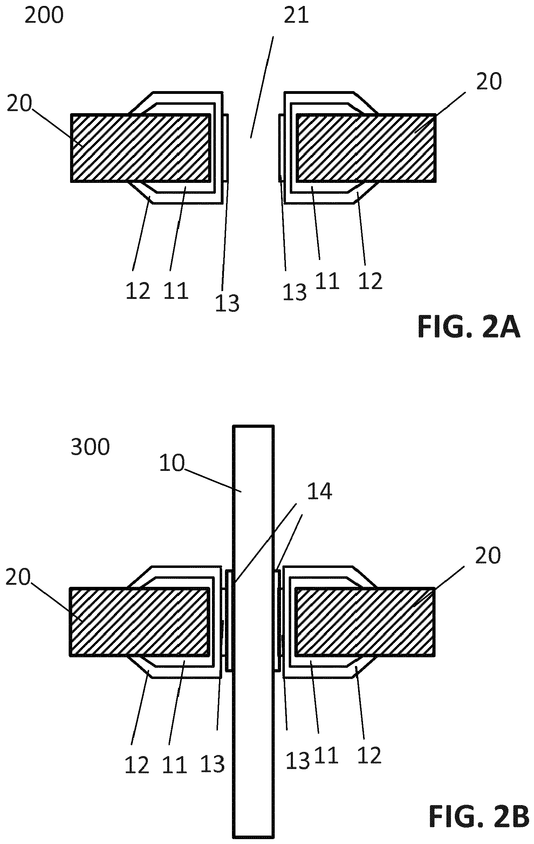

Fig. 2a shows an alternative embodiment of feedthrough component 200 of the invention. The alternative feedthrough component comprises an electrically conductive flange 20 with at least one opening 21, wherein a plasma ceramic layer 11 is disposed on the flange 10, particularly on the surface of the flange 10 surrounding the at least one opening 21. Also, in this alternative, the plasma ceramic layer 11 is covered by a gas-tight second layer 12, which is in turn at least partly covered by a third metal layer 13. Preferably, the flange is made of titanium or a titanium alloy.

Fig. 2b shows the mounting condition of the alternative feedthrough component, wherein a connecting pin 10 made of, for example, niobium, is joined to the alternative feedthrough component 200 of the invention. More specifically, the connecting pin 10 is brazed into the at least one opening 21, preferably via the third layer 13 and a suitable solder 14 such as a tin, silver or gold solder.

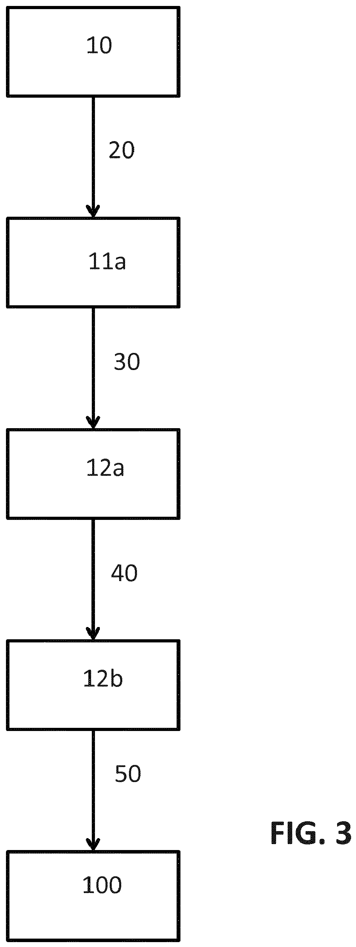

Fig. 3 illustrates one embodiment of the manufacturing method of the invention. First a rod-shaped connecting pin made of niobium is provided and flame of plasma sprayed with oxidic ceramic powder. During flame or plasma spraying the oxidic ceramic powder is heated up to the melting point of the ceramic, preferably with a water-stabilized plasma torch, and melted particles are propelled by the plasma jet towards the connecting pins, which termini have been masked or covered to leave them uncovered by the plasma ceramic 11. The resulting plasma ceramic layer 11 surrounds the lateral surface of the connecting pin 10 and has preferably a thickness in the range of 150 µm to 300 µm.

Since flame or plasma sprayed ceramics are insufficiently gas-tight, the plasma ceramic coated connecting pin 11a, particularly the plasma ceramic layer 11, is sealed with a glass or ceramic solder. For this, the plasma ceramic layer 11 is sprayed with or dived into an aqueous suspension comprising a ceramic oxide or glass powder and heated to thermally burn-in the components of the suspension. The resulting gas-tight, plasma ceramic coated pin 12a is coated with niobium, preferably by physical vapour deposition, and the suchlike coated pin 12b is gold brazed into a metal flange 20 as depicted in Fig. 1b.

Alternatively, the metal flange 20 of the feedthrough of the invention, particularly the openings 21 in the flange 20 configured to receive the connecting pins 10, may be flame or plasma sprayed and sealed in the above mentioned manner to yield a metal flange 200 with the above described first 11 and second layer 12 and optionally third layer 13.

Advantageously, flame or plasma sprayed ceramic layers 11 are thermally stable up to 1300 °C, possess outstanding insulating properties and tolerate rapid temperature shift much better than bulk ceramic materials. The present invention enables the design of much more stable flanges with larger welding ribs to reduce thermal tensions caused by welding of the flange into the housing. Furthermore, the risk of thermal warping is significantly reduced. Furthermore, the present invention further enables a reduction of the pitch distance and the component size. Also due to the enhanced stability of the plasma ceramic layer in comparison with common ceramic bulk bodies, the rejection rate may be significantly lowered.

In addition, the manufacturing method of the invention can be standardized. For example, for different types of feedthroughs the same connecting pins may be coated in the above named manner and stored, while only the flange has to be adapted to the intended design of the feedthrough.

## Claims

1. Feedthrough (300) for an electric component or electric device, particularly an implantable medical device, comprising at least one electrically conductive connecting element (10, 100) and an electrically conductive flange (20, 200) with at least one passage (21) configured to receive said at least one conductive connecting element (10, 100), wherein
- said at least one electrically conductive connecting element (10,100) or said electrically conductive flange (20, 200) comprises a first layer (11) made of a thermally spray ceramic, particularly a plasma ceramic, and a gas-tight second layer (12) made of glass or ceramic, and
- said first layer (11) is at least partly, particularly completely, surrounded by said second layer (12).

2. The feedthrough (300) according to claim 1, wherein said at least one electrically conductive connecting element (10,100) is arranged, particularly fixed, in said at least one passage (21) of said electrically conductive flange (20, 200).

3. The feedthrough (300) according to claim 1 or 2, wherein said second layer (12) is at least partly covered by a third layer (13) made of metal, wherein particularly said third layer comprises or consists of Nb, Pt, Pt/Ir, FeNi, FeNiCo, FeCr, Ta, Mo, W, Cr, Co, V, Ti or an alloy thereof.

4. The feedthrough (300) according to any one of the preceding claims, wherein said thermally sprayed ceramic (11), particularly said plasma ceramic, consists of or comprises Al₂O₃, Al₂TiO₅, TiO₂, MgO, CaO, Na₂O, K₂O, Nb₂O₅, ZrO₂, ZrO₂*SiO₂, Y₂O₃, 3Al₂O₃*2SiO2, 2Al₂O₃*SiO₂, MgAl₂O₄ or a mixture thereof.

5. The feedthrough (300) according to any one of the preceding claims, wherein said second layer (12) consists of or comprises SiO₂, ZrSiO₂, Na₂O, K₂O, Al₂O₃, MgO, CaO, BaO or a mixture thereof, particularly SiO₂-B₂O₃-Na₂O-K₂O-Al₂O₃-MgO-CaO.

6. The feedthrough (300) according to any one of the preceding claims, wherein said second layer (12) is **characterized by** an amorphous structure or a crystalline structure.

7. The feedthrough (300) according to any one of the preceding claims, wherein said at least one electrically conductive connecting element (10,100) consists of or comprises a metal or metal alloy, particularly Nb, Pt, Pt/Ir, FeNi, FeNiCo, FeCr, Ta, Mo, W, Cr, V, Ti or an alloy thereof, particularly Ti₆Al₄V.

8. The feedthrough (300) according to any one of the preceding claims, wherein said electrically conductive flange (20, 200) is made of a biocompatible material, particularly a biocompatible metal or metal alloy, more particularly titanium or a titanium alloy.

9. The feedthrough (300) according to any one of the preceding claims, wherein said first layer (11) is **characterized by** a thickness in the range of 100 µm to 500 µm, particularly in the range of 150 µm to 300 µm.

10. An electric device, particularly an implantable medical device, comprising a feedthrough (300) according to any one of the preceding claims.

11. An electric component, particularly a battery, a capacitor or a printed circuit board, comprising a feedthrough (300) according to any one of claims 1 to 9.

12. A method for manufacturing a feedthrough, particularly a feedthrough (300) according to any one of claims 1 to 9, wherein the method comprises the steps of:
- providing an electrically conductive connecting element (10) or an electrically conductive flange (20) with at least one passage (21) configured to receive said electrically conductive connecting element,
- applying (20) a ceramic powder, particularly an oxidic ceramic powder, on a surface portion of said electrically conductive connecting element (10) or said electrically conducting flange (20) by thermal spraying, particularly flame or plasma spraying, thereby yielding a thermally sprayed ceramic layer (11), particularly a plasma ceramic layer,
- applying (30) a suspended solution comprising a glazing component selected from a ceramic oxide or a glass former, and
- heating (40) said electrically conductive connecting element (10) or said electrically conductive flange (20) to or above the melting temperature of said glazing component, thereby yielding a gas-tight glaze layer (12).

13. The method according to claim 12, further comprising the step of:
- applying (50) a metal layer (13) at least on a part of said glaze layer (12).

14. The method according to claim 12 or 13, further comprising the step of:
- joining said electrically conductive connecting element (10, 100) to the electrically conductive flange (20, 200), wherein said electrically conductive connecting element (100, 10) is arranged within said at least one passage (21) of the electrically conductive flange (20, 200).

15. The method according to any one of claims 12 to 14, wherein said suspended solution further comprises one or more crystal nuclei.
